# EUROPEAN PATENT APPLICATION

(11) **EP 2 175 265 A1**
(43) Date of publication of application: **14.04.2010**
(21) Application number: 08166083.9
(22) Date of filing: 08.10.2008
(51) Int. Cl.: G01N 27/12

(54) **Hydrogen sensor and production method thereof**

(71) Applicant: IEE International Electronics & Engineering S.A.R.L., 6468 Echternach (LU)
(72) Inventor: Wittkowski, Thomas, 54411, Hermeskeil (DE)
(74) Representative: Office Freylinger

(57) **Abstract**

Producing a tunneling-based hydrogen sensor (12) comprises forming a first (18) and a second (20) electrode at a distance from one another on an electrically insulating surface of a substrate (16) and applying a hydrogen sensing layer (22) on that surface. Either the forming or the application step includes bringing the hydrogen sensing layer (22) into contact with the first (18) and second (20) electrodes. The application of the hydrogen sensing layer (22) includes printing of a layer of functional ink onto the electrically insulating surface, the functional ink comprising a dispersion of nanosized platinum group metal particles in a solvent, and curing the layer of functional ink by heating it in such as way as to cause at least part of the platinum group metal particles to form conductive paths having tunneling gaps therein. The invention further relates to a hydrogen sensor so produced.

## Description

### Technical field

The present invention generally relates to sensing of hydrogen in air, in particular to the detection of hydrogen levels exceeding critical thresholds.

### Background Art

Because of the high flammability of hydrogen gas (H₂) in air, there is a need for detecting hydrogen at levels below its lower explosive limit (about 4% at 25°C). A conventional approach to detect H₂ is based upon the resistance increase of palladium (Pd) taking place when hydrogen diffuses into the metal and thereby gives rise to palladium hydride having higher volume and lower conductivity than pure palladium.

More recently, hydrogen sensors have been developed, which use so-called palladium nanowires or mesowires (F. Favier et al., "Hydrogen Sensors and Switches from Electrodeposited Palladium Mesowire Arrays", Science 293, p. 2227-2231, 2001). In sensors of this kind, the nanowires form the main conduction paths between two contacts and contain gaps that progressively close as the volume of the palladium crystals of the nanowires increases upon exposure to hydrogen. The resistance of the conduction paths is dominated by the resistance at the gaps, which represent tunnel junctions. As a result, the overall electrical resistance of between the contacts decreases with increasing hydrogen partial pressure and provides a measure for that pressure.

In paper "A Hydrogen Sensor Based on Tunneling Between Palladium Clusters", Appl. Phys. Lett. 91, 181910 (2007), J. van Lith et al. present hydrogen sensors produced by depositing palladium clusters between two NiCr/Au contacts on a Si₃N₄ substrate until cluster coverage on the substrate surface is close below the percolation threshold. The gold contacts were applied using photolithography and thermal evaporation of 5 nm NiCr and 40 nm Au, followed by a lift-off process. The palladium clusters were deposited from a magnetron sputtering source with mean diameters between 3.5 and 6 nm, while the resistance between the contacts was measured. The deposition was stopped as soon as a predetermined resistance value was reached.

The above-described technique of producing tunneling-based hydrogen sensors is cost-intensive and currently not suitable for mass production of such sensors.

### Technical problem

It is an object of the present invention to reduce production costs of tunneling-based hydrogen sensors. This object is achieved by a method as claimed in claim 1 and a hydrogen sensor as claimed in claim 10.

### General Description of the Invention

The method of producing a tunneling-based hydrogen sensor comprises forming a first and a second electrodes at a distance from one another on an electrically insulating surface of a substrate (preferably a polymer film) and applying a hydrogen sensing layer on that surface. Depending on the order of application of the electrodes and the hydrogen sensing layer, either the one or the other of these steps includes bringing the hydrogen sensing layer into contact with the first and second electrodes. According to an aspect of the invention, the application of the hydrogen sensing layer includes printing of a layer of functional ink onto the electrically insulating surface, the functional ink comprising a dispersion of (preferably spherical) nanosized platinum group metal particles in a solvent, and curing the layer of functional ink by heating it in such as way as to cause at least part of the platinum group metal particles to form conductive paths (chains or clusters of particles) having tunneling gaps therein. Heat may be applied, for instance, by UV- or IR-radiation, microwave radiation, by induction and/or by energy transfer from hot air or another hot gas. As used herein, the term "nanosized" is used to qualify particles (or clusters) having a diameter of not more than 100 nm. After printing (which may comprise inkjet, gravure, flexo, offset, pad or screen printing), the morphology of the sensing material is tuned by heating in order to remove solvent(s) (e.g. water, polar or non-polar organic solvent, or the like) and organic additives present in the functional ink. Preferably, an external magnetic field having its field vector lying in the plane of the substrate and from the first to the second electrode (or vice versa) is applied before and/or while the functional ink is cured. Thereby, a magnetic moment is induced in the platinum group metal particles, which promotes the formation of chains of such particles in direction of the field vector. Most preferably, the magnetic field is kept upright until the platinum group metal particles have become substantially immobile on the substrate.

As those skilled will appreciate, the present method does not require deposition of platinum group metal clusters under vacuum conditions (as do chemical or physical vapor deposition techniques), which makes production easier and cheaper. Another benefit of the present method is that sensor resistance as well as the morphology of the hydrogen sensing layer are highly reproducible. The distance between the electrodes is preferably selected in the range from a few ten to a few hundred micrometers. The electrodes are preferably printed but they may also be prepared from gas phase with subsequent photolithography. In principle, the electrodes can be made of any conducting material but copper, gold or silver is currently preferred.

Preferably, the layer of functional ink has a thickness of approximately one monolayer of the nanosized platinum group metal particles. In this case, "approximately one" is to be understood as "at most three", preferably less and most preferably "at most one".

Advantageously, the dispersion may comprise a surfactant (e.g. tetralkylammonium salt or tetradodecylammonium nitrate) or a polymeric stabilizer, such as e.g. polyvinylpyrrolidone or polystyrene sulfonate, to stabilize, i.e. to avoid uncontrolled aggregation of the nanosized platinum group metal particles in the dispersion. Such surfactant or stabilizer particles form envelopes around the nanosized platinum group metal particles, which keep the latter at a distance from one another in the dispersion as well as in the dried but not yet fully cured state of the layer of functional ink.

The area density of nanosized platinum group metal particles in the layer of functional ink is preferably selected higher than the mathematical percolation threshold in two dimensions (which is between 42 and 47%). This may be surprising at first sight but it can be explained by the fact that the platinum group metal particles are normally not distributed homogeneously in a single layer but are clustered or stacked on one another for statistical and/or energetic reasons. Moreover, particles enveloped with surfactant or stabilizer are separated by minimum distance of twice the thickness of the envelopes, which amounts typically to between about 0.5 and about 3 nm in average. Preferably, within the boundaries of the dry layer of functional ink the surface coverage of the substrate by the nanosized platinum group metal particles amounts to between 42 and 75%.

Advantageously, the coalescence into conductive paths is controlled by measuring the electric resistance of the layer of functional ink and adjusting the heating depending on the measured electric resistance. Adjusting the heating may e.g. comprise stopping the heating as the resistance decreases below a predefined threshold. If the electrodes are applied before the functional ink, the resistance may be measured between them. Otherwise, the resistance could be measured by temporarily applying electric contacts to the layer of functional ink. It should be noted that the resistance could be measured on a test layer of functional ink printed next to the layer that is going to be part of the hydrogen sensor.

The size distribution of the platinum group metal particles is preferably such that at least 90% of the particles have diameters of at most 50 nm (abbreviated as D90 ≤ 50 nm); more preferably at least 90% of the particles have diameters of at most 25 nm (D90 ≤ 25 nm); even more preferably, at least 90% of the particles have diameters of at most 25 nm (D90 ≤ 25 nm) while at least 50% of the particles have diameters of at most 10 nm (D50 ≤ 10 nm).

The platinum group metal particles preferably consist of a metal selected from the platinum group (i.e. ruthenium, rhodium, palladium, osmium, iridium and platinum), an alloy of at least two metals selected from the platinum group or an alloy of a metal selected from the platinum group and a metal not belonging to the platinum group. As diffusion of hydrogen into palladium exceeds that of the other metals and the price of palladium compared to most of the other platinum group metals is relatively low, the platinum group particles are preferably palladium particles, i.e. particles consisting of elemental palladium or a palladium alloy (such as e.g. Pd-Ag, Pd-Pt, etc.).

According to a preferred aspect of the invention, the layer of functional ink is ink-jet printed onto the electrically insulating surface. As will be appreciated, ink-jet printing has several benefits, such as, for instance, (i) high cleanliness, (ii) printing of exact amounts of ink (accuracy down to the picoliter range), (iii) high positioning accuracy with respect to placing ink drops at the exact positions between the electrodes. In consequence, only a minimum amount of (expensive) Pd is required to achieve high H₂ sensitivity as well as highest reproducibility of the sensor response.

A hydrogen sensor produced according to the method described hereinbefore comprises a substrate having an electrically insulating surface, a first and a second electrode arranged at a distance from one another on that surface and a hydrogen sensing layer arranged in contact with the first and second electrodes. The hydrogen sensing layer comprises a cured layer of functional ink containing platinum group metal particles that form conductive paths having tunneling gaps therein.

The substrate could be glass or silicon with an SiO₂ surface but, preferably, the substrate is a flexible polymer film, e.g. a polyimide (PI) film, a polyether imide (PEI) film, a polyethylene naphtalate (PEN) film, a polycarbonate (PC) film, a polyethylene terephtalate (PET) film, a polymer-coated metal (e.g. aluminum) foil, an invar foil coated with an electrically insulating layer, a filled polymer film (whose filler is a dielectric material with low coefficient of thermal expansion), or a composite film comprising more than one of the cited films. Preferably, the substrate has a coefficient of linear thermal expansion of not more than 6.0·10⁻⁵ K⁻¹. In this case, it is assured that the resistance variation of the hydrogen sensor due to the presence of H₂ in the relevant pressure range is distinctly higher than the resistance change caused by temperature variations or externally applied mechanical strains. For instance, the absorption of hydrogen in palladium increases the lattice constant of the palladium nanoparticles by up to 2.5 %. Resistance variation caused by temperature-induced strain of a PI substrate (having a linear thermal expansion coefficient of 2.0·10⁻⁵ K⁻¹) is approximately one order of magnitude smaller than the resistance variation caused by a H₂ concentration varying in the range of about 1 to 5 % even if the temperature should vary between -40 and 105 °C. Therefore, whereas prior art hydrogen sensor had to be kept at a constant temperature, with the present sensor it is sufficient compensate for temperature changes. An example as to how such compensation may be carried out is indicated in the detailed description.

Those skilled will appreciate that there are no strict limitations with respect to the thickness of the substrate other than that the substrate must be suitable for printing on it. Typically, substrate thickness will amount to between 5 µm and 250 µm if a flexible polymer film is used. It shall be noted that substrate bending will lead to strains in the sensor and have an influence on the widths of the tunnel junctions between the platinum group metal particles and chains. Therefore, sensor bending should be taken into account when the hydrogen sensor is calibrated.

The response of the hydrogen sensor is affected by temperature changes, as the volume of the platinum group metal particles varies as a function not only of hydrogen partial pressure but also of temperature. A possible way to eliminate temperature effects is to actively keep the hydrogen sensor at a given temperature during the measurement. It should be noted that a hydrogen sensor according to the present invention is perfectly compatible for such use. According to a preferred aspect of the invention a hydrogen sensing device is proposed that comprises one or more hydrogen sensors as presented hereinbefore and at least one temperature sensor. Such temperature sensor may be used for compensating temperature-dependency of the hydrogen sensor as an alternative to keeping the temperature constant. In principle, any suitable production method could be considered for the temperature sensor; preferably, however, it is also provided by printing. The temperature sensor preferably comprises at least two electrodes (herein referred to as the third and fourth electrodes for distinction from the electrodes of the hydrogen sensor) arranged at a distance from one another on the electrically insulating surface and a temperature sensing layer arranged in contact in contact with the electrodes on the electrically insulating surface. The temperature sensing layer preferably has an electrical resistance that changes as a function of temperature, e.g. diluted silver, carbon black, a doped ceramic, material comprising carbon nanotubes, etc. If the hydrogen sensing device comprises more than one hydrogen sensor, the measurements thereof may be combined in a suitable way to increase measurement accuracy (e.g. by averaging or exclusion of outliers). Likewise, if the hydrogen sensing device comprises more than one temperature sensor, the measurements thereof may also be combined in a suitable way to yield a more accurate temperature value.

Another preferred aspect of the invention concerns a system for distributed sensing of hydrogen, comprising a plurality of hydrogen sensors and/or hydrogen sensing devices as described hereinbefore.

Those skilled will appreciate that the present invention bears the potential to reduce the costs of hydrogen sensors (by at least one order of magnitude compared to today's sensors) and pave the way to mass production of such sensors. Concerns with the reliability of such mass-produced sensors might be mitigated by using arrays of sensors to confer redundancy to the measurements and/or by more frequent replacement of the sensors. It is currently expected that even in combination with such service option, the sensors according to the present invention still present a significant economical benefit over today's sensors.

### Brief Description of the Drawings

Further details and advantages of the present invention will be apparent from the following detailed description of not limiting embodiments of the invention with reference to the attached drawings, wherein:
Fig. 1 is a schematic perspective view of a hydrogen sensing device equipped with a temperature sensor;
Fig. 2 is a schematic illustration of a distributed hydrogen sensing system.

### Description of Preferred Embodiments

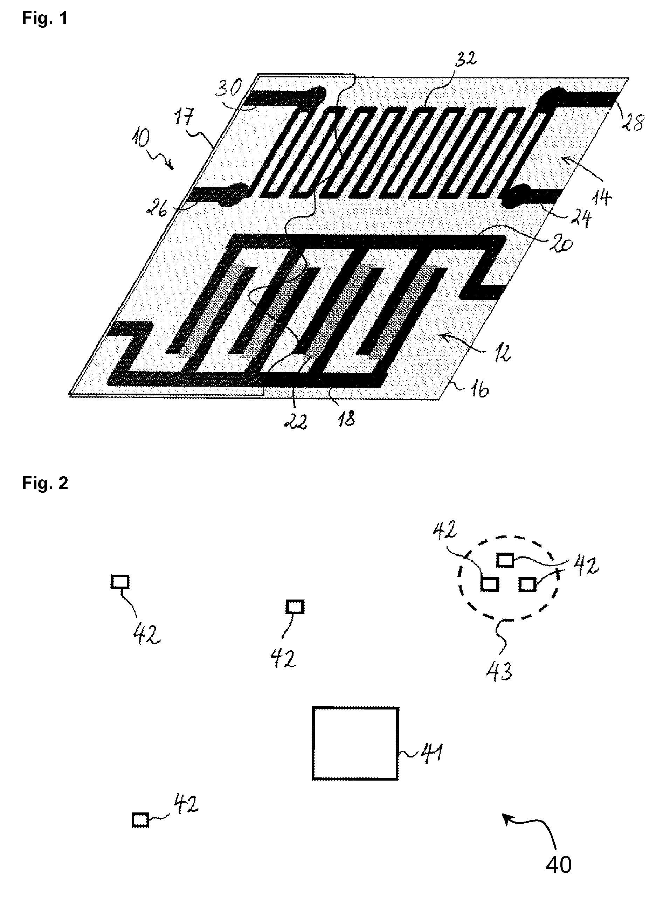

Fig. 1 shows a hydrogen sensing device 10 comprising a hydrogen sensor 12 and a temperature sensor 14. In the shown embodiment, hydrogen sensor 12 and temperature sensor 14 share a common substrate in form of a flexible polymer film 16. A dielectric protective film 17 (shown only in part) is arranged on top of the hydrogen and temperature sensors 12, 14. Protective film 17 preferably consists of a thin polymeric layer (e.g. of PMMA, PI, parylene, or the like), which may be laminated or printed onto the polymer film 16 and the hydrogen and temperature sensors 12, 14. The protective film 17 provides protection against mechanical strains as well as migration or corrosion over sensor lifetime. It should be noted that H₂ diffuses easily through a thin polymeric layer and that the response time of the hydrogen sensor is affected to a minor extent only.

Hydrogen sensor 12 comprises a first electrode 18 and a second electrode 20, arranged interdigitating with one another on the substrate 16 in such a way that the minimum distance between the electrode fingers of the first electrode and those of the second electrode amounts to between 10 and 100 µm. The lateral sizes of the electrodes 18, 20 may range from a few hundred micrometers up to a few millimeters. (It should be noted that the drawing is not to scale for sake of clarity.) The electrodes 18, 20 are preferably printed (preferably ink-jet printed) onto the substrate 16 using highly conductive ink, e.g. silver ink.

A hydrogen sensing layer 22 is disposed on the substrate 16 in such a way that it is in contact with the electrode fingers and bridges the gap between the fingers of the two electrodes 18, 20. Hydrogen sensing layer 22 is in this case formed by applying functional ink onto the desired locations of the substrate and taking care that the layer of functional ink overlaps with the fingers of the electrodes 18, 20. The functional ink that is used is a dispersion of nanosized palladium particles in a solvent, wherein the palladium particles are stabilized by polymer envelopes in order to avoid that they precipitate and/or agglomerate into larger clusters. Stabilized palladium nanoparticle dispersions are commercially available, e.g. from Meliorum Technologies (mean Pd particle diameter: about 10 nm; Pd concentration: 0.2 wt%; liquid carrier: either water or paraffin oil (CAS 8012-95-1) as can be purchased in a low-viscosity version e.g. from J.T. Baker). Due to the relatively low Pd concentration, the thickness of the wet print may be significantly higher than the print when dried: the wet print thickness may amount to tens of micrometers, whereas the dry print thickness is typically 1 to 3 monolayers of Pd particles.

After the functional ink has been applied on the predetermined locations in a (preferably ink-jet) printing step, a magnetic field is generated whose field vector parallel to the substrate is oriented substantially orthogonal to the electrode fingers (i.e. substantially along the desired direction of chain alignment) and the functional ink is cured by heating. During the heating, which is carried out under precisely determined conditions, first the solvent and then the stabilizing polymers evaporate. Subsequently, the morphology of the palladium particles and the open pore volume is caused to gradually change. In particular, unlike the solvent and the stabilizing polymers, the palladium particles remain on the substrate and are caused to form conductive chains of particles (by coalescence). During this step, the resistance of the hydrogen sensing layer is continuously monitored, e.g. by connecting a current source between the first and second electrodes 18, 20 and measuring the resulting voltage. When the resistance drops below a certain predefined (finite) threshold (e.g. about 125 Ω for a 8-nm-Pd-particles print having a length of 8 mm in the direction of conduction and 0.08 mm in the transverse direction), heating is stopped. This threshold is determined in such a way that each of the conductive paths formed between the first and second electrodes 18, 20 by the chains of particles still contains at least one tunnel junction, i.e. a small gap between chains of particles that charge carriers can only cross by quantum-mechanical tunneling. For practical reasons, the resistance of the hydrogen sensing layer is preferably chosen substantially (preferably orders of magnitude) higher than the resistance of the electrodes and any leads. It should be noted that the heating has to be carried out at temperatures compatible with the chosen substrate. Preferably, the temperature is selected in the range up to about 350°C (more preferably in the range up to about 250°C) and the duration of the heating step up to about 20 minutes. It should be noted that the higher the curing temperature is selected, the shorter will be the heating time interval (for instance: about 3 minutes at 350°C instead of about 15 minutes at about 250°C).

When the hydrogen sensor 12 is operating, it utilizes the strength of the electron tunneling effect (affecting and measurable through the sensor's DC resistance), which depends upon the hydrogen content in the surrounding gas atmosphere. The electrical resistance of palladium particles that are in contact with one another is low compared to the resistance though gaps between such chains of particles. The overall resistance between the electrodes of the hydrogen sensor is thus dominated by the contribution of the tunnel junctions. The mean width of the gaps between neighboring particles or chains of particles typically amounts to less than a few nanometers (e.g. less than 2 nm, preferably less than 1 nm), depending on the threshold resistance chosen for stopping the heating. (As the resistance through a tunnel junction increases exponentially with increasing gap width, the resistance turns out to be impracticably high if the overall effective width of the gaps is higher than about 5 nm.) Upon application of an electric field between the electrodes, charge carriers, in occurrence electrons, tunnel across the tunnel junction predominantly in one direction. By driving a predetermined current through the electrodes or applying a predetermined voltage between them, the resistance between the electrodes can be determined from the resulting voltage or current, respectively.

As indicated hereinbefore, palladium has an outstanding ability to absorb hydrogen (up to about 900 times its own volume). Absorption of hydrogen leads to an increase of the nanoparticle lattice constant. Thus, particle diameter increases with hydrogen concentration. This in turn leads to a reduction of the spacings between the particles and particle chains and thereby to a strong decrease of the tunneling resistance. In summary, the resistance of the hydrogen sensing layer decreases with increasing hydrogen content. Since particles are nanosized, the response time of the sensor is very fast (down to a few tens of milliseconds). The advantage of this concept over other existing sensor concepts is mainly its fast response, simplicity of sensor construction, and straightforward signal detection.

The temperature sensor 14 is preferably prepared via (ink-jet) printing. The temperature sensor of the shown embodiment of the hydrogen sensing device comprises four contact electrodes 24, 26, 28, 30 and a winding 32 of conductive material having a temperature-dependent resistance. The resistance of the winding 32 may be determined between the contact electrodes 28 and 30 preferably using a four-terminal configuration (also known as Kelvin connection). The winding is preferably made of a continuous silver print, which remains essentially unaffected by H₂ partial pressure because of the lower solubility of H₂ in this material and because of the absence of tunneling junctions that would influence the measurement. (ink-jetted) silver prints initially comprise nanosized particles. When the print is heated to temperatures that are compatible with the substrate, the silver particles are caused to coalesce into a continuous and compact silver layer without tunneling contacts and with a low resistivity. By ink-jet printing the temperature sensing element, one may achieve precise metering and positioning of the ink. The print morphology is precisely tuned during the subsequent temperature treatment. Optionally, the resistance of the winding 32 is measured in-situ during the heating, which may be stopped as soon as a pre-defined resistance value is reached. In this way, temperature sensors may be produced with very small tolerances. Preferably, the production of a hydrogen sensing device comprising a printed hydrogen sensor and a printed temperature sensor starts with the application of the electrodes and leads of both sensors (e.g. by printing) and then continues with the printing and the curing (e.g. by heating) of the temperature sensing layer. Subsequently, the functional ink should be applied and cured as indicated hereinbefore. The reason for this order of steps is that the conductive nanoparticles of the temperature-sensitive ink have to be allowed to form a continuous and compact layer which requires normally longer heating and at higher temperatures than the curing of the hydrogen sensing layer. Therefore, the subsequent applying and curing of the hydrogen sensing layer does not significantly affect the properties of the temperature sensing layer.

Before the hydrogen sensing device 10 of Fig. 1 can be used, sensor calibration has to be carried out. This includes, as a first step, determining the resistance of the temperature sensor element, denoted herein as Rₜₑₘₚ(T), as a function of temperature. The temperature range over which the calibration has to be carried out depends on the intended application of the hydrogen sensing device. For automotive applications, the calibration range is typically from -40 to 105 °C. Due to the high reproducibility of the temperature sensor, the calibration curve Rₜₑₘₚ(T) is characteristic for all produced temperature sensors of a certain type (same sensor configuration). This means that Rₜₑₘₚ(T) does not need to be determined for every single hydrogen sensing device but may be determined once for a given batch. The validity of the temperature curve is preferably checked at regular intervals to assure production quality. It should be noted that the recorded temperature curve Rₜₑₘₚ(T) is an effective temperature curve, i.e. it comprises the intrinsic effect of resistance increase in metals as a function of temperature as well as the thermal expansions of the metal and the substrate.

Sensor calibration further includes recording a characteristic set of resistance curves of the hydrogen sensor as a function of H₂ concentration and temperature. The measured response of the hydrogen sensor is preferably the relative resistance change, denoted herein ΔR/R0, where ΔR = R0-R_{H2}, R0 is the resistance of the hydrogen sensor when no H₂ is present (e.g. in air because the natural H₂ concentration in air is negligibly small) and at a predetermined temperature and R_{H2} is the resistance of the hydrogen sensor for a certain amount of H₂ in the surrounding atmosphere. For the determination of the characteristic set of curves, the temperature is varied in the desired temperature range (e.g. as above between -40 and 105 °C) and the H₂ concentration in air is varied in the desired concentration range (e.g. between 0 and 5 % by volume). For each given temperature (and thus for each value Rₜₑₘₚ), ΔR/R0 is a monotonously increasing function of the H₂ concentration. In consequence, the inverse problem, i.e. the determination of the characteristic set of curves for the H₂ concentration, denoted herein as Conc_{H2}(ΔR/R0, Rₜₑₘₚ), can be solved unambiguously.

The set of characteristic curves does not need to be determined for every single hydrogen sensing device but may be determined once for a given production batch. Low production tolerances ensure that the characteristic set of curves ΔR/R0(Conc_{H2}, Rₜₑₘₚ) and the curve Rₜₑₘₚ(T) are valid for all H2 sensing devices of the same type. To improve sensing accuracy, the resistance of every produced temperature sensor may be measured in air (ConC_{H2}≈0) and at a defined temperature T0. A possible deviation of the so measured resistance, R_{temp,measured}(T0) from the temperature curve Rₜₑₘₚ(T) at T=T0 will be small so that a shift of the calibration curve provides a suitable correction for this particular temperature sensing element. Thus R_{temp,cal}(T) = Rₜₑₘₚ(T) + ΔRₜₑₘₚ, where R_{temp,cal}(T) denotes the calibrated temperature curve for a particular temperature sensor, and ΔRₜₑₘₚ = R_{temp,measured}(T0) - Rₜₑₘₚ(T=T0). The resistance R0 (resistance at T=T0 and ConC_{H2}≈0) of a H₂ sensor may possibly deviate slightly from the value adjusted in the production process. In order to take such deviations into account, one preferably measures R0 for every produced H₂ sensor and stores this value of R0 so that later computation of ΔR/R0 is as accurate as possible. Optionally also, the temperature curve of a temperature sensor may be calibrated by interpreting resistance measurements performed at several temperatures, i.e. at T0, T1, T2, etc. Optionally, the above described calibration routine is performed after the hydrogen sensing device is mounted where it should later operate, provided that, at the moment of the calibration, the environment is free of H₂ and the temperature is well defined.

As will be appreciated, by using the relative resistance change ΔR/R0 (instead of the absolute resistance value) of the hydrogen sensor, the relative change of the widths of the tunnel junctions rather than their absolute values determines the outcome of the measurement. Thus by measuring R0 in the same bending conditions as those in which the hydrogen sensor is to be used, the influence of bending is automatically compensated for. For a 25 µm thick substrate, bending strains in an outer fiber are about one order of magnitude smaller than thermal strains provided that the bending radius is not below 10 cm. This property allows mounting a flexible hydrogen sensor fabricated according to the present method on moderately curved surfaces, e.g. by means of a double-faced adhesive tape (possibly applied to the hydrogen sensor or the hydrogen sensing device during production).

Fig. 2 shows a schematic of a system 40 for distributed sensing of hydrogen comprising a plurality of hydrogen sensors and/or hydrogen sensing devices 42 mounted in a space such as a fuel cell vehicle or in a room containing devices that are prone to H₂ leakage. The system device comprises a control station 41, which is in communication with the sensors 42 and which receives the measurement data from them. As shown in the dashed circle 43, some of the hydrogen sensors are arranged as a group or array in a single location. Others are arranged individually in the space to be monitored. Whether a single sensor or a plurality thereof is used in a particular location depends on the redundancy and confidence level aimed at. The system may be configured such that the hydrogen sensors do not receive control signals from the control station but only forward their measurements. Preferably, however, control station 41 is configured such that it can actively communicate with the hydrogen sensors and/or the temperature sensors, for instance to initiate and/or stop the measurements. Most preferably, the control station is configured to address each hydrogen sensor and/or temperature sensor individually. The measurement values (which can be raw resistance values or derived H₂ concentrations and/or temperature values) collected by each sensor are transmitted to the control station. If raw resistance values are transmitted, the control station must contain the necessary electronics to calculate the hydrogen level or temperature for each sensor. In any case, the control station then decides, based upon the determined H₂ concentration values, whether the H2 level is within a safe range or exceeds a predefined threshold. The control station may then initiate appropriate actions, e.g. closing of valves, generating warning signals, disrupting of the electrical power supply, etc. The rate at which measurement data may be acquired and evaluated is mainly limited by the response time of the H₂ sensors. As this response time is typically below 100 ms, a measurement rate of 10 Hz or more is possible.

According to a first embodiment of the system 40, the hydrogen sensors or hydrogen sensing devices are wired with the control station via an analog or digital interface. Each sensor may thus be powered by the control station via the wire connection.

According to a second embodiment of the system 40, the control station and the sensors communicate wirelessly, e.g. by a radio link, an IR communication interface or any other interface for wireless data transfer. In this case, each pad may be equipped with a battery or another energy storage component (e.g. a capacitor). Alternatively, powering of the sensors is effected via the wireless communication link. Transmitted energy then has to be buffered on in the hydrogen sensor or hydrogen sensing device for the measurement and the subsequent data transfer to the control station, e.g. In a battery or a capacitor. Most preferably, the energy storage or buffering component is printed on the same substrate as the hydrogen sensor.

## Claims

1. Method of producing a hydrogen sensor, said method comprising:
provision of a substrate having an electrically insulating surface;
formation of a first and a second electrode at a distance from one another on said electrically insulating surface;
application of a hydrogen sensing layer on said electrically insulating surface, wherein at least one of said formation of the first and second electrodes and said application of the hydrogen sensing layer includes bringing said hydrogen sensing layer into contact with said first and second electrodes;
said method being **characterised in that** said application of the hydrogen sensing layer comprises printing of a layer of functional ink onto said electrically insulating surface, said functional ink comprising a dispersion of nanosized platinum group metal particles in a solvent, and curing said layer of functional ink by heating it in such as way as to cause at least part of said platinum group metal particles to form conductive paths having tunneling gaps therein.

2. The method according to claim 1, wherein said layer of functional ink has a thickness of approximately one monolayer of said nanosized platinum group metal particles.

3. The method according to claim 1 or 2 wherein said layer of functional ink has an area density of nanosized platinum group metal particles higher than the mathematical percolation threshold in two dimensions.

4. The method according to any one of claims 1 to 3, wherein said coalescence into conductive paths is controlled by measuring an electric resistance of said layer of functional ink and adjusting said heating depending on said measured electric resistance.

5. The method according to claim 4, wherein said heating is stopped as said resistance decreases below a predefined threshold.

6. The method according to any one of claims 1 to 5, wherein said platinum group metal particles a size distribution such that at least 90% of the particles have diameters of at most 50 nm.

7. The method according to any one of claims 1 to 6, wherein said platinum group metal particles consist of a metal selected from the platinum group, an alloy of at least two metals selected from the platinum group or an alloy of a metal selected from the platinum group and a metal not belonging to the platinum group.

8. The method according to any one of claims 1 to 7, wherein said dispersion comprises at least one of a surfactant and a polymeric stabilizer to stabilize said nanosized platinum group metal particles in said dispersion

9. The method according to any one of claims 1 to 8, wherein said layer of functional ink is ink-jet printed onto said electrically insulating surface.

10. A hydrogen sensor comprising
a substrate having an electrically insulating surface;
a first and a second electrode arranged at a distance from one another on said electrically insulating surface;
a hydrogen sensing layer arranged in contact with said first and second electrodes on said electrically insulating surface,
**characterized in that** said hydrogen sensing layer comprises a cured layer of functional ink comprising platinum group metal particles that form conductive paths having tunneling gaps therein.

11. The hydrogen sensor according to claim 10, wherein said substrate has a coefficient of linear thermal expansion of not more than 6.0 10⁻⁵ K⁻¹.

12. The hydrogen sensor according to claim 8, wherein said substrate comprises at least one of a polymer film, a filled polymer film, a polymer-coated metal foil, and an invar foil.

13. Hydrogen sensing device, comprising a hydrogen sensor as claimed in any one of claims 10 to 12 and at least one temperature sensor.

14. The hydrogen sensing device according to claim 13, wherein said temperature sensor comprises a third and a fourth electrodes arranged at a distance from one another on said electrically insulating surface and a temperature sensing layer arranged in contact in contact with said third and fourth electrodes on said electrically insulating surface.

15. System for distributed sensing of hydrogen, comprising a plurality of hydrogen sensors according to any one of claims 10 to 12 and/or hydrogen sensing devices according to any one of claims 13 to 14.
